# EUROPEAN PATENT SPECIFICATION

(11) **EP 4 105 334 B1**
(45) Date of publication and mention of the grant of the patent: **14.05.2025**
(21) Application number: 22757468.8
(22) Date of filing: 07.01.2022
(51) Int. Cl.: A01K 67/0276, C07K 14/51, C12N 15/90, C12N 15/113

(54) **INTERMUSCULAR BONE-FREE CRUCIAN STRAIN AND CULTIVATION METHOD THEREFOR**
KARAUSCHE (CARASSIUS AURATUS)-STAMM OHNE INTERMUSKULÄRE GRÄTEN UND VERFAHREN ZU SEINER ZÜCHTUNG
SOUCHE DE CARASSIN COMMUN SANS OS INTERMUSCULAIRE ET SON PROCÉDÉ DE CULTURE

(30) Priority: 30.04.2021 CN 202110478410
(43) Date of publication of application: 21.12.2022
(73) Proprietor: Heilongjiang River Fisheries Research Institute of Chinese Academy of Fishery Sciences, Harbin, Heilongjiang 150070 (CN)
(72) Inventor: KUANG, Youyi, Harbin, Heilongjiang 150070 (CN); ZHENG, Xianhu, Harbin, Heilongjiang 150070 (CN); TONG, Guangxiang, Harbin, Heilongjiang 150070 (CN); SUN, Zhipeng, Harbin, Heilongjiang 150070 (CN); CAO, Dingchen, Harbin, Heilongjiang 150070 (CN); YAN, Ting, Harbin, Heilongjiang 150070 (CN); XU, Huan, Harbin, Heilongjiang 150070 (CN); DONG, Le, Harbin, Heilongjiang 150070 (CN); YANG, Xiaoxing, Harbin, Heilongjiang 150070 (CN); LIU, Tianqi, Harbin, Heilongjiang 150070 (CN); ZHANG, Tan, Harbin, Heilongjiang 150070 (CN); ZHANG, Tingting, Harbin, Heilongjiang 150070 (CN); SUN, Xiaowen, Harbin, Heilongjiang 150070 (CN)
(74) Representative: RGTH
(86) International application number: PCT/CN2022/070608
(87) International publication number: WO 2022/227692

(56) References cited:
- CN-A- 110 684 777
- CN-A- 110 684 777
- CN-A- 112 772 468
- CN-A- 113 151 361
- NIE CHUN-HONG ET AL: "Understanding the development of intermuscular bones in teleost: status and future directions for aquaculture", REVIEWS IN AQUACULTURE, vol. 12, no. 2, May 2020 (2020-05-01), pages 759 - 772, XP093082933, ISSN: 1753-5123, Retrieved from the Internet <URL:https://onlinelibrary.wiley.com/doi/full-xml/10.1111/raq.12348> DOI: 10.1111/raq.12348
- KUANG YOUYI ET AL: "Generate a new crucian carp (Carassius auratus) strain without intermuscular bones by knocking out bmp6", AQUACULTURE, vol. 569, May 2023 (2023-05-01), Amsterdam, NL, pages 739407, XP093082820, ISSN: 0044-8486, DOI: 10.1016/j.aquaculture.2023.739407
- ERICKSON PRISCILLA A, BAEK JIYEON, HART JAMES C, CLEVES PHILLIP A, MILLER CRAIG T: "Genetic Dissection of a Supergene Implicates Tfap2a in Craniofacial Evolution of Threespine Sticklebacks", GENETICS, vol. 209, no. 2, 1 June 2018 (2018-06-01), pages 591 - 605, XP055981818, DOI: 10.1534/genetics.118.300760
- ZHANG WEI-ZHUO, LAN TIAN, NIE CHUN-HONG, GUAN NING-NAN, GAO ZE-XIA: "Characterization and spatiotemporal expression analysis of nine bone morphogenetic protein family genes during intermuscular bone development in blunt snout bream", GENE, ELSEVIER AMSTERDAM, NL, vol. 642, 1 February 2018 (2018-02-01), NL , pages 116 - 124, XP055981820, ISSN: 0378-1119, DOI: 10.1016/j.gene.2017.11.027
- NIE CHUNHONG, WAN SHIMING, CHEN YULONG, ZHU DEJIE, WANG XUDONG, DONG XIAORU, GAO ZE-XIA: "Loss of scleraxis leads to distinct reduction of mineralized intermuscular bone in zebrafish", AQUACULTURE AND FISHERIES, vol. 6, no. 2, 1 March 2021 (2021-03-01), pages 169 - 177, XP055981822, ISSN: 2468-550X, DOI: 10.1016/j.aaf.2020.04.006
- SMITH, A. AVARON, F. GUAY, D. PADHI, B.K. AKIMENKO, M.A.: "Inhibition of BMP signaling during zebrafish fin regeneration disrupts fin growth and scleroblast differentiation and function", DEVELOPMENTAL BIOLOGY, ELSEVIER, AMSTERDAM, NL, vol. 299, no. 2, 28 October 2006 (2006-10-28), AMSTERDAM, NL , pages 438 - 454, XP005735534, ISSN: 0012-1606, DOI: 10.1016/j.ydbio.2006.08.016
- MA QIAN; FAN YANJUN; ZHUANG ZHIMENG; LIU SHUFANG: "Cloning, expression profiling and promoter functional analysis of bone morphogenetic protein 2 in the tongue sole (Cynoglossus semilaevis)", ACTA OCEANOLOGICA SINICA, THE CHINESE SOCIETY OF OCEANOGRAPHY, BEIJING, vol. 37, no. 2, 8 February 2018 (2018-02-08), Beijing, pages 76 - 84, XP036427311, ISSN: 0253-505X, DOI: 10.1007/s13131-018-1164-x
- YANG JIAN, TONG GUANGXIANG ; ZHENG XIANHU; SUN ZHIPENG; LU WEIHUA ; SUN XIAOWEN: "Comparative Analysis of Skeletal Development between Wildtype Zebrafish and Intermuscular Bone-Deficient Mutants", SHUISHENG-SHENGWU-XUEBAO = ACTA HYDROBIOLOGICA SINICA, vol. 44, no. 5, 31 May 2020 (2020-05-31), CHINA , pages 546 - 553, XP055981846, ISSN: 1000-3207, DOI: 10.7541/2020.067

## Description

### TECHNICAL FIELD

The present disclosure relates to a reagent for the provision of *Carassius auratus* strain without intermuscular bones, belonging to the technical field of aquaculture.

### BACKGROUND ART

Cyprinid is one of the main aquaculture fish in China, with an annual output of about 20 million tons in China, providing nearly 1/3 of the high-quality animal protein for the Chinese people. However, because of lots of intermuscular bones in cyprinid fishes, it is inconvenient for people to eat, and may even lead to physical injuries such as getting stuck in the throat. At the same time, the processing of fish products (such as fish balls, etc.) is also hindered. Therefore, the genetic improvement of intermuscular bones in cyprinid fishes has become one of the most important targets of aquaculture genetic breeding.

At present, there are some reports on selection and breeding of intermuscular bones. For example, Xiao-feng Xu *et al.*^{[1]} found a normally grown and developed intermuscular bone-deficient mutant in the gynogenetic group of grass carp, but no mutant population of grass carp with intermuscular bone-deficient or less intermuscular bone was obtained as reported. Brazilian scientists^{[2]} screened a population lacking intermuscular bones in *Colossoma macropomum* using X-rays, but no population with less intermuscular bone or intermuscular bone-deficient in the subsequent breeding program was obtained^{[3]}. Guo *et al.*^{[4]} conducted a hybrid experiment between *Megalobrama terminalis9and Culter alburnus ♂,* and showed that the number of intermuscular bones (127) in the hybrid progeny of *Megalobrama terminalis and Cutter alburnusowas* smaller than that of the male parent (137), greater than that of the female parent (124), but significantly fewer than the number of intermuscular bones in the hybrid progeny (133) of *Megalobrama amblycephala ♀* and *Cutter alburnus ♂,* which indicated that varieties with less intermuscular bones could be obtained by hybrid breeding methods, and an invention patent (publication number of CN107347747A) was applied then. Li Ling *et al*.^{[5]} reported that the number of intermuscular bones in the artificial cultivation of hybrid *Carassius auratus* was less than that of the wild *Carassius auratus,* and the number of intermuscular bones in the *Carassius auratus* could be reduced by artificial cultivation and hybrid breeding. Bao-long Bao *et al.* invented a method for thicking the intermuscular bones by knocking out the *MSTN* gene^{[9-12]}. By knocking out the *scxa* gene in zebrafish (*Danio rerio*), Gao Zexia *et al.* obtained mutants with more than 70% reduction of the number of intermuscular bones, but also caused dysplasia of other bones such as ribs^{[6-8]}, and the applications in other Cyprinoid fish has not been reported. In conclusion, although there are some methods to reduce the number of intermuscular bones in cyprinid fish, there is still no new variety/strain without intermuscular bones.

Crucian carp (*Carassius auratus*) is one of the main aquaculture varieties in China, with an annual production of about 3 million tons. Its meat is delicious and popular among people. At the same time, the quality of *Carassius auratus* has been affected due to the intermuscular bones (the average number of intermuscular bones in different varieties of *Carassius auratus* is about 71 to 84), thus, intermuscular bones are also an important target for their genetic breeding. Although it has been reported that the number of intermuscular bones in *Carassius auratus* can be reduced by hybrid breeding and artificial selection, no strain or variety with more than 50% reduction in the number of intermuscular bones has been found.

### References

[1]Xu, X., Zheng, J., Qian, Y. & Luo, C. Normally grown and developed intermuscular bone-deficient mutant in grass carp, Ctenopharyngodon idellus. Chin Sci Bulletin Chin Version 60, 52 6 (2015).
[2]Perazza, C. A. et al. Lack of intermuscular bones in specimens of Colossoma macropomum: An unusual phenotype to be incorporated into genetic improvement programs. Aquaculture 472, 57 60 (2017).
[3]Stokstad, E. Tomorrow's catch. Science 370, 902-905 (2020).
[4]Guo, H.-H. et al. Comparative analysis of the growth performance and intermuscular bone traits in F1 hybrids of black bream (Megalobrama terminalis) (♀) × topmouth culter (Culter alburnus) (♂). Aquaculture (2018) doi:10.1016/j.aquaculture. 2018.03.037.
[5]Li, L. et al. Comparative analysis of intermuscular bones in fish of different ploidies. Sci China Life Sci 56, 341-350 (2013).
[6]Nie, C. et al. Loss of scleraxis leads to distinct reduction of mineralized intermuscular bone in zebrafish. Aquac Fish 6, 169-177 (2021).
[7]Kague, E. et al. Scleraxis genes are required for normal musculoskeletal development and for rib growth and mineralization in zebrafish. Faseb J 33, 9116-9130 (2019).
[8]CN110684777A (Huazhong Agricultural University, Publication date 2020.01.14) ;
[9]CN111560401A (Shanghai Ocean University, Publication date 2020.08.21) ;
[10]CN111549030A (Shanghai Ocean University, Publication date 2020.08.18) ;
[11]CN111549031A (Shanghai Ocean University, Publication date 2020.08.18) ;
[12]CN111500581A (Shanghai Ocean University, Publication date 2020.08.07) ;
[13]Chen, Z. et al. De novo assembly of the goldfish (Carassius auratus) genome and the evolution of genes after whole-genome duplication. Sci Adv 5, eaav0547 (2019).
[14]Tong, G. et al. De novo assembly and characterization of the Hucho taimen transcriptome. Ecol Evol 8, 1271 1285 (2018).
[15]Fiume, M. et al. CRISPResso2 provides accurate and rapid genome editing sequence analysis. Nat Biotechnol 37, 224-226 (2019).

### SUMMARY

In order to perform genetic improvement of intermuscular bones in *Carassius auratus,* the present disclosure proposes a reagent for knocking out the bmp6 genes in Carassius auratus genome, the reagent comprising sgRNA forward primers and sgRNA reverse primers designed for knockout target sites of the bmp6 genes as described in claim 1.

The present application further provides, as an example not falling into the scope of the present invention, a method for breeding *Carassius auratus* with fewer intermuscular bones or without intermuscular bones by knocking out the *bmp6* gene.

The method for breeding *Carassius auratus* strains without intermuscular bones of the present disclosure carries out the breeding of the *Carassius auratus* strain without intermuscular bones according to the following steps:

Designing knockout target sites for two copies of *bmp6* gene in the *Carassius auratus* genome, *bmp6a* and *bmp6b,* respectively, and then obtaining F2 generation individuals from a homozygous line with *bmp6a* and *bmp6b* double gene mutation through two rounds of gene knockout and screening, and then using the F2 generation individuals from the homozygous line with *bmp6a* and *bmp6b* double gene mutation to propagate to form a new *Carassius auratus* strain without intermuscular bone;

Among them, mixing the sgRNA corresponding to *(bmp6a), (bmp6b)* or *(bmp6a* and *bmp6b)* with Cas9 protein and microinjecting a mixture obtained into *Carassius auratus* embryos in the single cell stage, to perform the first round of gene knockout to construct the F0 generation population, culturing the F0 generation population for 3 to 5 months followed by PIT labeling and DNA extraction, then sequencing to determine the alleles and mutation rates of somatic mutations of *Carassius auratus,* selecting the F0 generation individuals with the somatic mutation rate of more than 95% as parents to prepare 0-generation fertilized eggs;

Mixing the sgRNAs corresponding to *(bmp6a), (bmp6b)* or *(bmp6a* and *bmp6b)* with Cas9 protein and microinjecting a mixture obtained into 0-generation fertilized eggs to perform the second round of gene knockout to construct the F1 generation population, culturing the F1 generation population for 3 to 5 months followed by PIT labeling and DNA extraction, then sequencing to determine the alleles and mutation rates of somatic mutations of *Carassius auratus,* selecting the F1 generation individuals from a somatic *bmp6a* and *bmp6b* double gene mutant line with the somatic mutation rate of more than 95% as parents for reproduction to construct F2 generation, then selecting F2 generation individuals from a homozygous line with *bmp6a* and *bmp6b* double gene mutation.

*Carassius auratus* varieties with less than 20 intermuscular bones and without intermuscular bones are obtained by the method of the present disclosure.

Since the *Carassius auratus* genome has undergone a fourth round of genome duplication event^{[13]}, there are two copies of the *bmp6* gene in the *Carassius auratus* genome.

### BRIEF DESCRIPTION OF THE DRAWINGS

Fig. 1 is a comparison picture of bone staining observation of a typical individual in Example 1, and arrows in the figure refer to intermuscular bones;
Fig. 2 is an X-ray diagram of wild-type *Carassius auratus* bone in Example 1, and the arrow in the figure indicates intermuscular bones;
Fig. 3 is an X-ray diagram of caudal bones of a mutant in Example 1, from which it can be seen that no intermuscular bone is present in the caudal muscle tissue;
Fig. 4 is an X-ray diagram of trunk bones of a mutant in Example 1, from which it can be seen that no intermuscular bone is present in back muscle tissue of trunk;
Fig. 5A, 5B and 5 C are diagrams showing the sequencing results of exon 1 of the new *Carassius auratus* strain *bmp6a* in Example 1; CAA-1, CAA-2, and CAA-3 are target sites of exon 1 of *bmp6a*; WT represents wild type, the number in sequence number is the PIT labeling number of mutant, and the letter followed by PIT labeling number is the label of mutant allele;
Fig. 6A, 6B and 6C are diagrams showing the sequencing results of exon 1 of the new *Carassius auratus* strain *bmp6b* in Example 1; CAA-7, CAA-8, and CAA-9 are target sites of exon 1 of *bmp6b*; WT represents wild type, the number in sequence number is the PIT labeling number of mutants, and the letter followed by PIT labeling number is the label of mutant allele;
Fig. 7 is a comparison diagram of exon 1 protein sequences of the new *Carassius auratus* strain *bmp6a* in Example 1; WT represents wild type, the number in sequence number is the PIT labeling number of mutants, and the letter followed by PIT labeling number is the label of mutant allele;
Fig. 8 is a comparison diagram of exon 1 protein sequences of the new *Carassius auratus* strain *bmp6b* in Example 1; WT represents wild type, the number in sequence number is the PIT labeling number of mutants, and the letter followed by PIT labeling number is the label of mutant allele;
Fig. 9 is a micro-CT image of bones of wild-type and the new *Carassius auratus* strain in Example 1;
Fig. 10 is a viviperception diagram by X-ray of wild type, typical individuals with few intermuscular bones and typical individuals without intermuscular bones in Example 1, arrows indicate intermuscular bones.

### DETAILED DESCRIPTION OF THE EMBODIMENTS

The present disclosure is further described in detail below in conjunction with examples. The following examples are intended to illustrate the present disclosure, but not to limit the scope herein.

The experimental methods used in the following examples are conventional methods unless otherwise specified. The materials, reagents, methods and instruments used, unless otherwise specified, are conventional materials, reagents, methods and instruments in the art, which are commercially available to those skilled in the art.

Embodiment 1: The breeding method of *Carassius auratus* strain without intermuscular bones of the present embodiment:
The target sites are designed to knock out two copies of *bmp6* gene in the *Carassius auratus* genome, *bmp6a* and *bmp6b,* respectively, and then F2 generation individuals from a homozygous line with *bmp6a* and *bmp6b* double gene mutation are obtained through two rounds of gene knockout and screening, and then the F2 generation individuals from the homozygous line with *bmp6a* and *bmp6b* double gene mutation are propagated to form a new *Carassius auratus* strain without intermuscular bone;
Among them, the sgRNAs corresponding to *(bmp6a), (bmp6b)* or *(bmp6a* and *bmp6b)* are mixed with Cas9 protein, and the obtained mixture is microinjected into *Carassius auratus* embryos in the single cell stage, the first round of gene knockout is performed to construct the F0 generation population, and then the F0 generation population is cultured for 3 to 5 months followed by PIT labeling and DNA extraction then the alleles and mutation rates of somatic mutations of *Carassius auratus* are determined by sequencing, and the F0 generation individuals with the somatic mutation rate of more than 95% are selected as parents to prepare 0-generation fertilized eggs;
The sgRNAs corresponding to *(bmp6a), (bmp6b)* or *(bmp6a* and *bmp6b)* are mixed with Cas9 protein, and the obtained mixture is microinjected into 0-generation fertilized eggs, the second round of gene knockout is performed to construct the F1 generation population, and then the F1 generation population is cultured for 3 to 5 months followed by PIT labeling and DNA extraction, then the alleles and mutation rates of the somatic mutations of *Carassius auratus* are determined by sequencing, and the F1 generation individuals from a somatic *bmp6a* and *bmp6b* double gene mutant line with the somatic mutation rate of more than 95% are selected as parents for reproduction to construct an F2 generation, then F2 generation individuals from a homozygous line with *bmp6a* and *bmp6b* double gene mutation are selected.

Among them, the DNA extraction and sequencing method is as follows: 100µL of lysis solution is added (the lysis solution comprises 0.5 mg/mL proteinase K, 10 mM Tris (pH 8.0), 50mM KCl, 0.3 %Tween 20, 0.3% NP40) to 0.3-0.5cm² fin clip samples; the lysis methods is: the PCR instrument is set at 55°C for 6h, 98°C for 10min for lysis; after lysis, the obtained product is mixed well, then centrifuged at 1000-2000 rpm for 2min, and the supernatant is taken as a PCR amplification template.

PCR amplification: 2 pairs of primers are used for PCR amplification, of which, 1 pair of primers is the target site primers (the primer sequences used to amplify the target fragment are shown in Table 2 and concatenated by the M13 universal primer sequence at 5'end), and the other pair of primers is index primers (the M13 universal primer sequence and the index base sequence at the 5' end used to identify the sample). A two-step PCR amplification is performed. The PCR amplification volume in the first step is 10 µL, including 1 µL of genomic DNA supernatant, 0.5 µL of 1 µM forward and reverse target primers, and 5µL of 2×Dream Taq Master Mix (Thermo Fisher, CA, USA), the volume is supplemented to 10 µL with enzyme-free water; PCR program: 95°C for 3min; 10 cycles of 95°C for 30s, 60°C for 30s and 72°C for 30s; 72°C for 2min; PCR amplification in step 2 is that 0.5 µL of 5 µM forward and reverse index primers are added to the PCR amplification product obtained in step 1, the PCR program is set to 95°C for 2 min; 6 cycles of 95°C for 30s, 58°C for 30s and 72°C for 30s; 15 cycles of 95°C for 30s, 72°C for 30s; 72°C for 2min.

After PCR amplification, the PCR products are mixed in equal amounts, and a DNA sequencing library is constructed with the TrueSeq Sample Preparation Kit, and is sequenced using Illumina MiSeq sequencing platform with 300bp Pair-End mode. The data obtained by high-throughput sequencing are first used to identify amplification loci and samples using the method described by Tong *et al.*^{[14]}. The sequenced data of each sample in each PCR product is analyzed using the CRISPResso2^{[15]} program to determine the alleles and mutation rates of somatic mutations.

Among them, Sanger sequencing is used for mutant detection: PCR amplification is performed on the samples using the primers shown in Table 2, and the volume is set to 25 µL: 2 µL of genomic DNA supernatant, 1 µL of 10 µM forward and reverse target primers, 12.5 µL of 2 × DreamTaq Master Mix (Thermo Fisher, CA, USA), the volume is supplemented to 25 µL with enzyme-free water; PCR program is 95°C for 3 min; 35 cycles of 95°C for 30s, 60°C for 30s, 72°C for 30s; 72°C for 5min. PCR products are detected by 1.5% agarose gel electrophoresis. After detection, the products are purified and recovered, TA clone is performed, 2 µL of PCR products are taken after colony PCR amplification, and detected with 8% polypropylene, and the colonies with different band sizes from the control are selected for Sanger sequencing.

Embodiment 2: The difference between this embodiment and Embodiment 1 is that the knockout target sites of the *bmp6* gene are shown in Table 1 in this embodiment.

**Table 1 Knockout target sites of bmp6 gene**

| Gene | sgRNA s Numb er | Genome coordinat es | Exon | Target sites sequence | sgRNA forward primer |
|---|---|---|---|---|---|
| *bmp6* a | CAA1 | NC_03926 6.1:57844 76 | Exon 1 | GGTAGCAGTTCTGCACT AGACGG (SEQ ID NO:1) | TTCTAATACGACTCACTATAGGT AGCAGTTCTGCACTAGAGTTTTA GAGCTAGA (SEQ ID NO:12) |
| | CAA2 | NC_03926 6.1:57845 23 | Exon 1 | CCAGCGGAGGTTGCGGA CTCAGG (SEQ ID NO:2) | TTCTAATACGACTCACTATAGCC AGCGGAGGTTGCGGACTCGTTT TAGAGCTAGA (SEQ ID NO:13) |
| | CAA3 | NC_03926 6.1:57845 62 | Exon 1 | GAAAGAGATTCTGTCCA TACTGG (SEQ ID NO:3) | TTCTAATACGACTCACTATAGGA AAGAGATTCTGTCCATAC GTTTTAGAGCTAGA (SEQ ID NO:14) |
| | CAA4 | NC_03926 6.1:58154 87 | Exon 2 | TCTTTATGGTGTCTTCTC TGTGG (SEQ ID NO:4) | TTCTAATACGACTCACTATAGTC TTTATGGTGTCTTCTCTGGTTTT AGAGCTAGA (SEQ ID NO:15) |
| | CAA5 | NC_03926 6.1:58155 27 | Exon 2 | GGCCTCTCCCTCTGGAAT CTGGG (SEQ ID NO:5) | TTCTAATACGACTCACTATAGGC CTCTCCCTCTGGAATCTGTTTTA GAGCTAGA(SEQ ID NO:16) |
| | CAA6 | NC_03926 6.1:58155 58 | Exon 2 | TGCAGAATTCAGGATCT ACAAGG (SEQ ID NO:6) | TTCTAATACGACTCACTATAGTG CAGAATTCAGGATCTACAGTTTT AGAGCTAGA (SEQ ID NO:17) |
| *bmp6 b* | CAA7 | NC_03929 1.1:81275 37 | Exon 1 | GGCAGGTAGTAGTTCTG TACTGG (SEQ ID NO:7) | TTCTAATACGACTCACTATAGGC AGGTAGTAGTTCTGTACGTTTTA GAGCTAGA (SEQ ID NO:18) |
| | CAA8 | NC_03929 1.1:81274 77 | Exon 1 | AGCCCAACTTCATTCATC GGAGG (SEQ ID NO:8) | TTCTAATACGACTCACTATAGAG CCCAACTTCATTCATCGGGTTTT AGAGCTAGA(SEQ ID NO:19) |
| | CAA9 | NC_03929 .1:812742 5 | Exon 1 | GAAAGAGATCCTGTCCA TACTGG (SEQ ID NO:9) | TTCTAATACGACTCACTATAGGA AAGAGATCCTGTCCATACGTTTT AGAGCTAGA (SEQ ID NO:20) |
| | CAA10 | NC_03929 .1:809726 4 | Exon 2 | GGCCTCTCCCTCTGGAAT CTGGG (SEQ ID NO:10) | TTCTAATACGACTCACTATAGGC CTCTCCCTCTGGAATCTGTTTTA GAGCTAGA (SEQ ID NO:21) |
| | CAA11 | NC_03929 1.1:80972 33 | Exon 2 | AGCAGAATTCAGGATCT ACAAGG (SEQ ID NO:11) | TTCTAATACGACTCACTATAGAG CAGAATTCAGGATCTACAGTTTT AGAGCTAGA (SEQ ID NO:22) |

Others are the same as Embodiment 1.

**Table 2 Primers for PCR amplification**

| Primer name | Amplifie d region | Forward primer sequence | Reverse primer sequence | Size of amplifie d fragmen t |
|---|---|---|---|---|
| CAA-imb1a-E1 | bmp6a exon1 | ATCACAGACTGGATGTAAA GATGA(SEQ ID NO:23) | CATGAAGAGGGGTGCT GAA (SEQ ID NO:24) | 250 bp |
| CAA-imb1a-E2 | bmp6a exon2 | GTTCGTCAAGCACACTAAA ACT (SEQ ID NO:25) | CTTTCTGGATGTTCCCC AA (SEQ ID NO:26) | 250 bp |
| CAA-imb1b-E1 | bmp6b exon1 | TCCAGCATGAAGAGGGGT G (SEQ ID NO:27) | CTGGATGTAAAGATGA CAAGCA (SEQ ID NO:28) | 247bp |
| CAA-imb1b-E2 | bmp6b exon2 | ACAGGCAGCAGTTGACTTG (SEQ ID NO:29) | GCTTTCACCACAGAGA AGACA (SEQ ID NO:30) | 322bp |

Embodiment 3: The difference between this embodiment and Embodiment 1 or 2 is that: the sgRNA forward primer shown in Table 1 and the sgRNA reverse primer with the sequence of 5'-GATCCGCACCGACTCGGTGCCACTTTTTCAAGTTGATAACGGACTAGCCTTATTTTAACTTGCTATTTCT AGCTCTAAAAC-3' (SEQ ID NO:31) are used to synthesize sgRNA in vitro. The sgRNA synthesis volume in vitro is: 2.5 µL of 10 µM sgRNA forward and reverse primers, 25 µL of 2×Dream Taq Master Mix (ThermoFisher, CA, USA), and sgRNA synthesis volume is supplemented to 50 µL with enzyme-free water; a total of 100 µL is amplified in 2 tubes. The sgRNA synthesis procedure in vitro is: denaturation at 95°C for 3 min, followed by 30 cycles of 95°C for 30s, 58°C for 30s, 72°C for 30s; extension at 72°C for 5min; then the obtained amplified product is purified and recovered, followed by an in vitro transcription, and 30 µL of reaction volume is established for each target site: 1 µg of sgRNA PCR recovered product, 10 µL of NTP Buffer Mix, 2 µL of T7 RNA Polymerase Mix, the reaction volume is supplemented to 30 µL with enzyme-free water; transcription was performed at 37°C for 4 h, 20 µL of enzyme-free water is added after the reaction, and 2 µL of DNase I was added after mixing. Digestion at 37°C for 15 min to remove DNA. Others are the same as the embodiments 1 or 2.

The in vitro PCR products of sgRNA are detected by 1.5% agarose gel electrophoresis, and the length of products is 120bp. After detection, the PCR products are purified and recovered with a PCR product purification and recovery kit (Exygen), and the concentration is determined with a Qubit 3 kit (Thermo Fisher, CA, USA) for use. The recovery concentration of sgRNA PCR products is in a range of 100 to 160 ng/µL.

The in vitro transcribed sgRNAs are purified and recovered with an RNA purification kit (Qiagen). The concentration of the recovered product is determined by using a Qubit 3 kit (Thermo Fisher, CA, USA) and the recovered product is stored in a -80 °C refrigerator until use. The recovery concentration of in vitro transcribed sgRNA is in a range of 800 to 3000 ng/µL.

Embodiment 4: The difference between this embodiment and Embodiment 1 or 2 or 3 is that the microinjection method in this embodiment is: the sgRNA synthesized in vitro and Cas9 protein (NEB M0646, MA, USA) are mixed in a molar ratio of 3:1, a mixture obtained is incubated at room temperature for 10 min, then 25% phenol red is added, and the obtained product is injected into *Carassius auratus* embryos in the single cell stage; among them, the target site sgRNAs on each exon are mixed in equal amounts and injected with a final concentration no less than 50ng/µL of each sgRNA, the control group is injected with 25% phenol red, and the injection volume of each fertilized egg is 1nL±0.02nL. Others are the same as the Embodiment 1, 2 or 3.

### Example 1

Breeding method of *Carassius auratus* strain without intermuscular bone:
The target sites were designed to knock out the two copies of *bmp6* gene in the *Carassius auratus* genome, *bmp6a* and *bmp6b,* respectively, and then F2 generation individuals from a homozygous line with *bmp6a* and *bmp6b* double gene mutation were obtained through two rounds of gene knockout and screening, and then the F2 generation individuals from the homozygous line with *bmp6a* and *bmp6b* double gene mutation were propagated to form a new *Carassius auratus* strain without intermuscular bones;
Among them, the sgRNAs corresponding to *(bmp6a), (bmp6b)* or *(bmp6a* and *bmp6b)* were mixed with Cas9 protein, and the obtained mixture was microinjected into *Carassius auratus* embryos in the single cell stage, the first round of gene knockout was performed to construct the F0 generation population, and then the F0 generation population was cultured for 3 to 5 months followed by PIT labeling and DNA extraction, then the alleles and mutation rates of somatic mutations of *Carassius auratus* was determined by sequencing, and the F0 generation individuals with the somatic mutation rate of more than 95% were selected as parents to prepare 0-generation fertilized eggs;
The sgRNAs corresponding to *(bmp6a), (bmp6b)* or *(bmp6a* and *bmp6b)* were mixed with Cas9 protein, and the obtained mixture was microinjected into the 0-generation fertilized eggs, the second round of gene knockout was performed to construct the F1 generation population, and then the F1 generation population was cultured for 3 to 5 months followed by PIT labeling and DNA extraction, then the alleles and mutation rates of the somatic mutations of *Carassius auratus* were determined by sequencing, and the F1 generation individuals from a somatic *bmp6a* and *bmp6b* double gene mutant line with the somatic mutation rate of more than 95% were selected as parents for reproduction to construct a F2 generation, then F2 generation individuals from a homozygous line with *bmp6a* and *bmp6b* double gene mutation were selected.

Among them, the DNA extraction and sequencing method was as follows: 100 µL of lysis solution was added (the composition of the lysate is 0.5mg/mL proteinase K, 10mM Tris (pH 8.0), 50mM KCI, 0.3% Tween20, 0.3% NP40) to 0.3-0.5cm² fin clip samples; the lysis methods was: the PCR instrument was set at 55°C for 6h, 98°C for 10min for lysis; after lysis, the obtained product was mixed well, then centrifuged at 1000-2000rpm for 2min, and the supernatant was taken as the PCR amplification template. PCR amplification: 2 pairs of primers were used for PCR amplification, of which, 1 pair of primers was the target site primers (the primer sequences used to amplify the target fragment as shown in Table 2 and the 5'-end M13 universal primer sequence), and the other pair of primers were index primers (the M13 universal primer sequence and the index base sequence at the 5' end used to identify the sample). PCR amplification was performed in 2 steps. The PCR amplification volume in the first step was 10 µL, including 1 µL of genomic DNA supernatant, 0.5 µL of 1 µM forward and reverse target primers, and 5µL of 2×Dream Taq Master Mix (Thermo Fisher, CA, USA), the volume was supplemented to 10µL with enzyme-free water; PCR program: 95°C for 3min; 10 cycles of 95°C for 30s, 60°C for 30s and 72°C for 30s; 72°C for 2min; PCR amplification in step 2 is that 0.5 µL of 5 µM forward and reverse index primers were added to the PCR amplification product obtained in step 1, the PCR program was set to 95°C for 2 min; 6 cycles of 95°C for 30s, 58°C for 30s and 72°C for 30s; 15 cycles of 95°C for 30s, 72°C for 30s; 72°C for 2min. After PCR amplification, the PCR products were mixed in equal amounts, and a DNA sequencing library was constructed with the TrueSeq Sample Preparation Kit, and was sequenced by using Illumina MiSeq sequencing platform with 300bp Pair-End mode. The data obtained by high-throughput sequencing were first used to identify amplification loci and samples using the method described by Tong *et al* (Tong *et al.* 2018). The sequenced data of each sample in each PCR product was analyzed using the CRISPResso2 (Fiume *et al.* 2019) program to determine the alleles and mutation rates of somatic mutations.

Among them, Sanger sequencing was used for mutant detection: PCR amplification was performed on the samples using the primers shown in Table 2, and the system was set to 25 µL: 2 µL of genomic DNA supernatant, 1 µL of 10 µM forward and reverse target primers, 12.5 µL of 2 × DreamTaq Master Mix (Thermo Fisher, CA, USA), the volume was supplemented to 25 µL with enzyme-free water; PCR program was 95°C for 3 min; 35 cycles of 95°C for 30s, 60°C for 30s, 72°C for 30s; 72°C for 5min. PCR products were detected by 1.5% agarose gel electrophoresis. After detection, the products were purified and recovered, TA clone was performed, 2 µL of PCR products were taken after colony PCR amplification, and detected with 8% polypropylene, and the colonies with different size bands from the control were selected for Sanger sequencing.

Among them, the knockout target sites of the *bmp6* gene were shown in Table 1.

The sgRNA forward primer was shown in Table 1 and the sgRNA reverse primer with the sequence of 5'-GATCCGCACCGACTCGGTGCCACTTTTTCAAGTTGATAACGGACTAGCCTTATTTTAACTTGCTATTTCT AGCTCTAAAAC-3' (SEQ ID NO:31) were used to synthesize sgRNA in vitro. The sgRNA synthesis volume in vitro was: 2.5 µL of 10 µM sgRNA forward and reverse primers, 25 µL of 2xDreamTaq Master mix (Thermo Fisher, CA, USA), the volume was supplemented to 50 µL with enzyme-free water; a total of 100 µL was amplified in 2 tubes. The sgRNA synthesis procedure in vitro was: denaturation at 95°C for 3 min, followed by 30 cycles of 95°C for 30s, 58°C for 30s, 72°C for 30s; extension at 72°C for 5 min; then the obtained amplified product was purified and recovered, followed by an in vitro transcription, and 30 µL of reaction volume was established for each target site: 1 µg of sgRNA PCR recovered product, 10 µL of NTP Buffer Mix, and 2 µL of T7 RNA Polymerase Mix, the volume was supplemented to 30 µL with enzyme-free water; transcription was performed at 37°C for 4 h, 20 µL of enzyme-free water was added after the reaction, and 2 µL of DNase I was added after mixing. Digestion at 37°C for 15 min to remove DNA.

In vitro sgRNA PCR products were detected by 1.5% agarose gel electrophoresis, and the length of products was 120bp. After detection, the sgRNA PCR products were purified and recovered with a PCR product purification and recovery kit (Exygen), and the concentration was determined with a Qubit 3 kit (Thermo Fisher, CA, USA) for use. The recovery concentration of the sgRNA PCR products was in a range of 100 to 160 ng/µL.

The in vitro transcribed sgRNAs were purified and recovered with an RNA purification kit (Qiagen). The concentration of the recovered product was determined by using a Qubit 3 kit (Thermo Fisher, CA, USA) and the recovered product was stored in a -80 °C refrigerator until use. The recovery concentration of in vitro transcribed sgRNA was in a range of 800 to 3000 ng/µL.

The microinjection method was as follows: sgRNA synthesized in vitro and Cas9 protein (NEB M0646, MA, USA) were mixed at a molar concentration ratio of 3:1, a mixture obtained was incubated at room temperature for 10 min, then 25% phenol red was added, and the obtained product was injected into *Carassius auratus* embryos in the single cell stage; among them, the target site sgRNAs on each exon were mixed in equal amounts and injected with a final concentration no less than 50ng/µL of each sgRNA, the control group was injected with 25% phenol red, and the injection volume of each fertilized egg is 1nL±0.02nL.

The diploid *Carassius auratus* used in this example came from the Hulan Experiment Station of the Heilongjiang River Fisheries Research Institute, Chinese Academy of Fishery Sciences. The parents fish were cultured in a 700m² fish pond, and were injected with oxytocin drugs (4 µg/kg luteinizing releasing hormone analogue, LRH-A2; 1 mg/k DOM, 100 units/kg chorionic gonadotropin HCG) for spawn. After artificial fertilization, the obtained fertilized eggs were incubated in a hatching tank of 24cm×20cm×24cm, and the hatching water temperature was 22°C-23°C. After hatching, larvae were fed with *Artemia salina* 4 times/d after the yolk was absorbed. The larvae were moved to an outdoor 500m² pond to culture when they were cultured in a hatching tank to the size of 2cm-3cm. Artificial diet was fed in the fish fingerling breeding stage, 2 times/d.

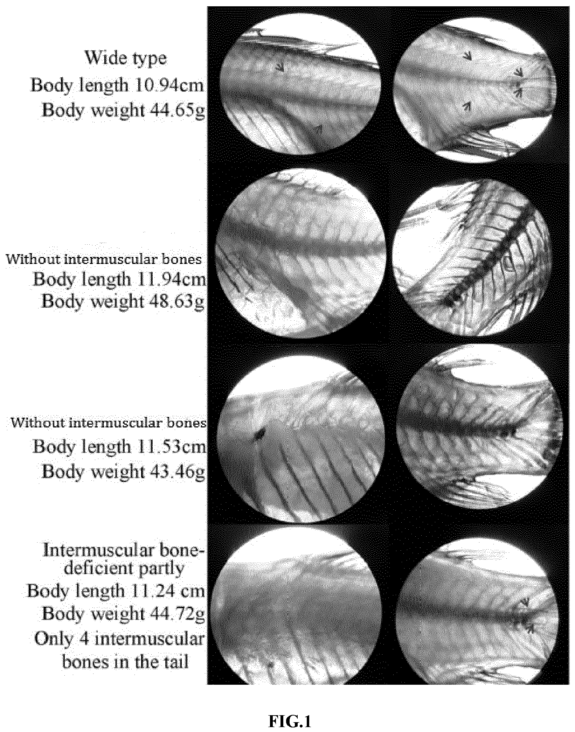

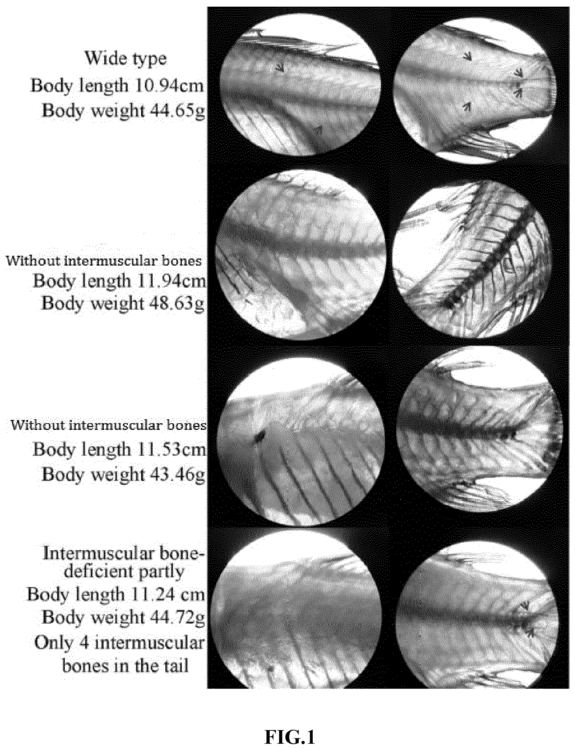

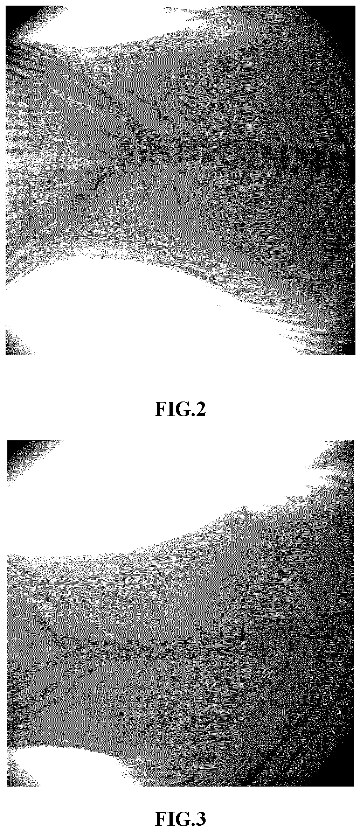

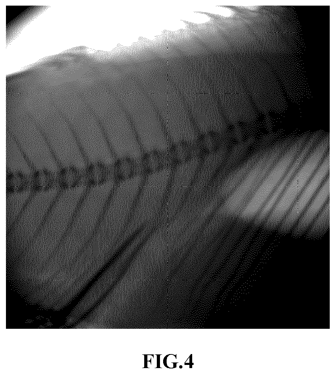

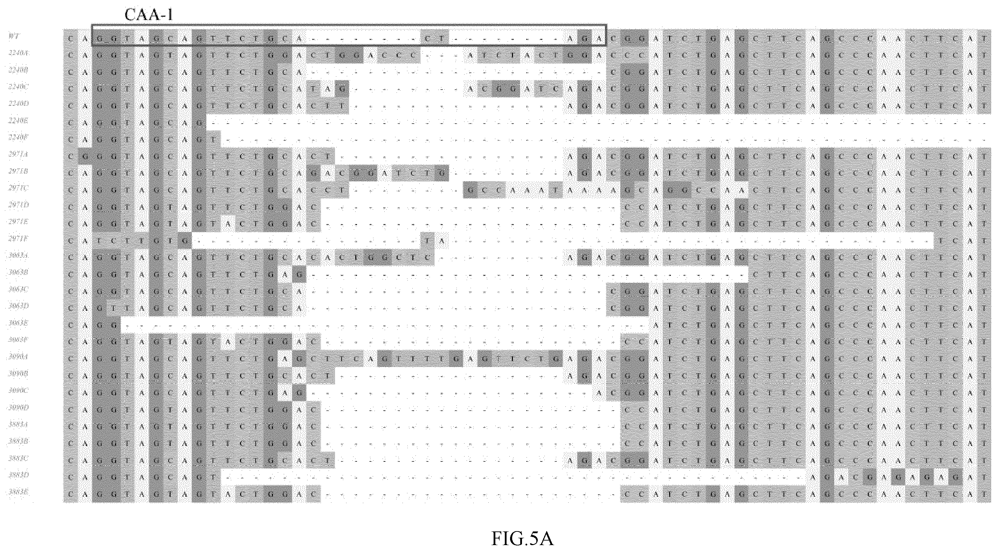

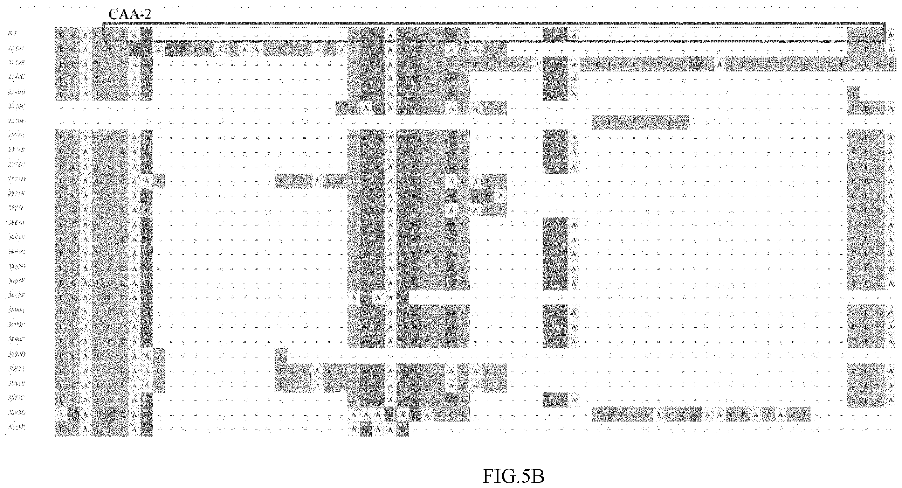

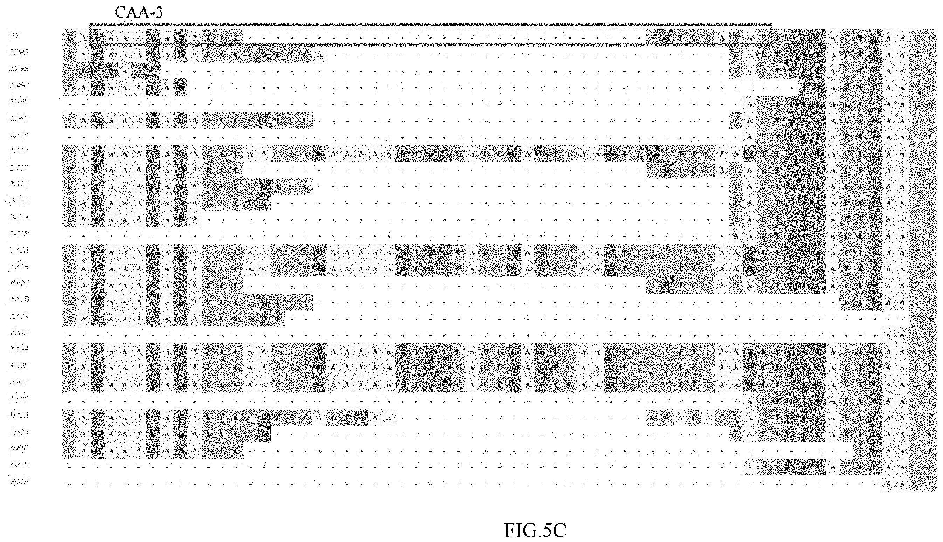

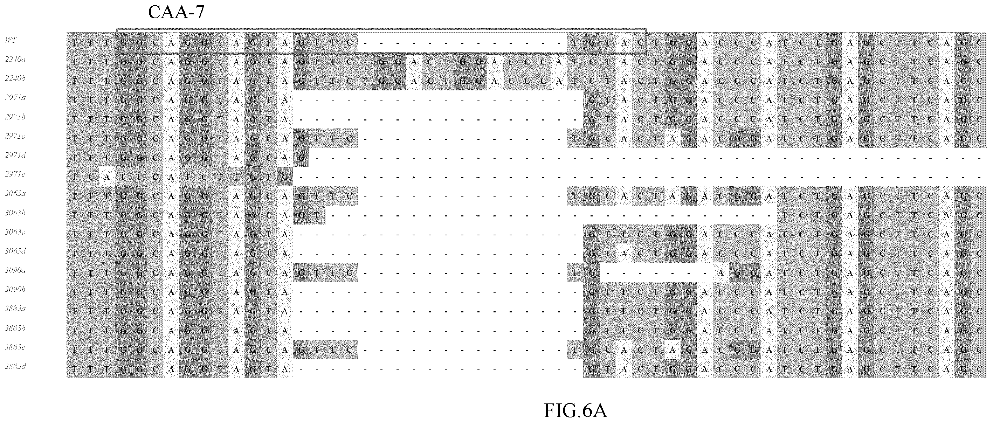

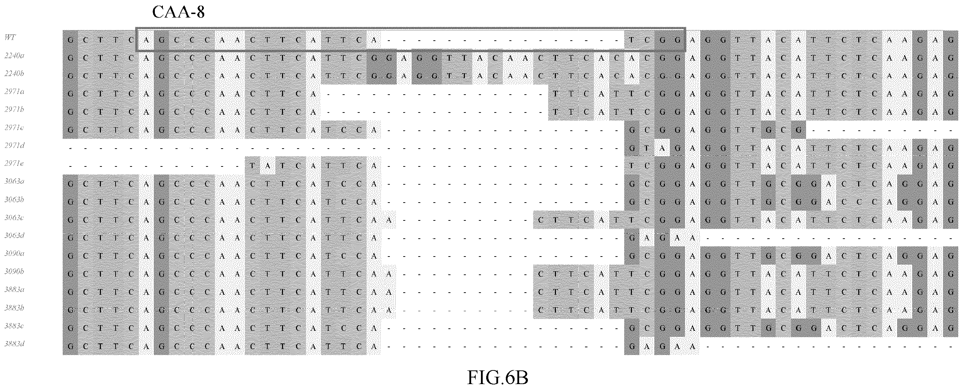

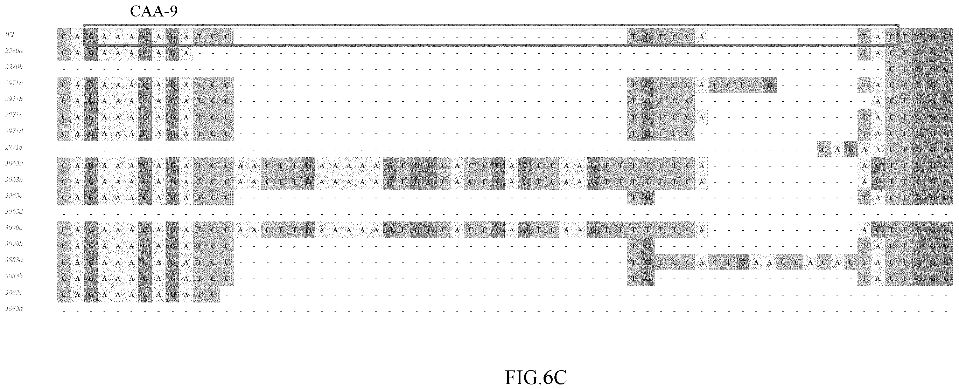

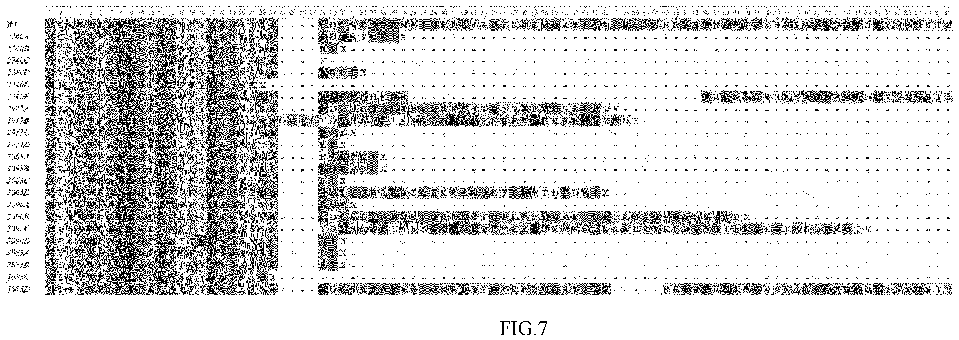

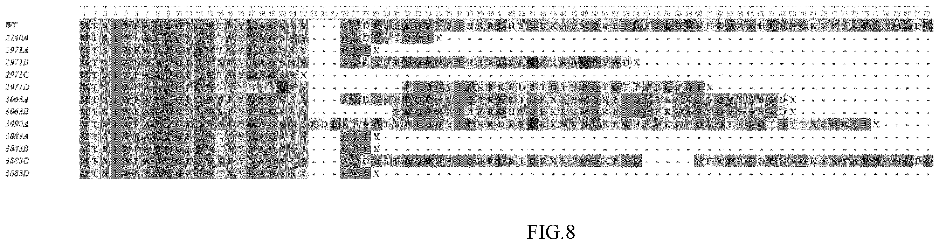

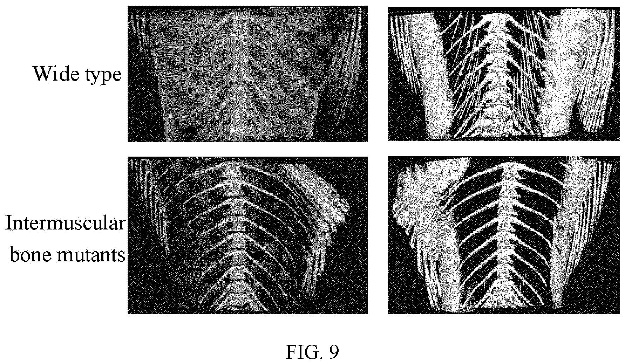

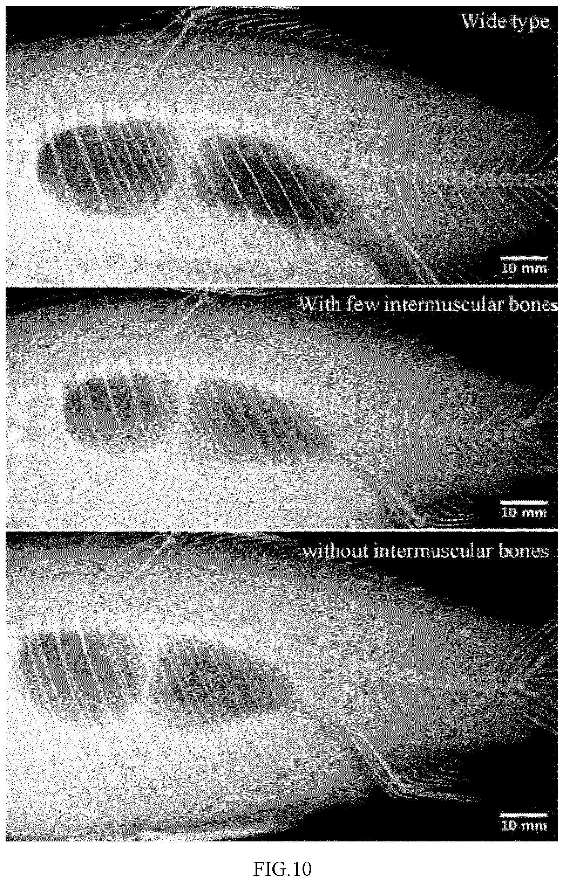

The bones of the new *Carassius auratus* strain with intermuscular bone-deficient in this example were observed by bone-staining, and it was found that the intermuscular bones were completely deficient in 5 samples, and the number of intermuscular bones in 4 samples was only 4 to 20 (as shown in Fig.1 to 4, 9 and 10). The mutant alleles of above samples in exon 1 of *bmp6a* and exon 1 of *bmp6b* were detected, and it was found that mutations were observed in all three target sites of exon 1 (as shown in Fig.5A to C and Fig.6A to C). Premature of stop codons in proteins was caused by gene knockout (as shown in Fig.7 and 8).

Although the present disclosure has been described in detail through the above example, it is only a part of embodiments of the present disclosure, rather than all embodiments. People can also obtain other embodiments according to the present embodiment without creativity.

## Claims

1. A reagent for knocking out *bmp6a* and *bmp6b* genes in *Carassius auratus* genome, the reagent comprising sgRNA forward primers and sgRNA reverse primers designed for knockout target sites of the *bmp6* genes; wherein the knockout target sites of the bmp6 genes are shown in Table 1,
| Table 1Gene | sgRNAs Number | Genome coordinates | Exon | Target sequence |
|---|---|---|---|---|
| *bmp6a* | CAA1 | NC_039266.1:578 4476 | Exon1 | GGTAGCAGTTCTGCA CTAGACGG |
| | CAA2 | NC_039266.1:578 4523 | Exon1 | CCAGCGGAGGTTGCG GACTCAGG |
| | CAA3 | NC_039266.1:578 4562 | Exon1 | GAAAGAGATTCTGTC CATACTGG |
| | CAA4 | NC_039266.1:581 5487 | Exon2 | TCTTTATGGTGTCTTCT CTGTGG |
| | CAA5 | NC_039266.1:581 5527 | Exon2 | GGCCTCTCCCTCTGGA ATCTGGG |
| | CAA6 | NC_039266.1:581 5558 | Exon2 | TGCAGAATTCAGGAT CTACAAGG |
| *bmp6b* | CAA7 | NC_039291.1:812 7537 | Exon1 | GGCAGGTAGTAGTTC TGTACTGG |
| | CAA8 | NC_039291.1:812 7477 | Exon1 | AGCCCAACTTCATTCA TCGGAGG |
| | CAA9 | NC_03929.1:8127 425 | Exon1 | GAAAGAGATCCTGTC CATACTGG |
| | CAA10 | NC_03929.1:8097 264 | Exon2 | GGCCTCTCCCTCTGGA ATCTGGG |
| | CAA11 | NC_039291.1:809 7233 | Exon2 | AGCAGAATTCAGGAT CTACAAGG |

2. The reagent according to claim 1, wherein the sgRNA forward primers are shown in following table,
| Gene | sgRNAs Number | sgRNA forward primer |
|---|---|---|
| *bmp6a* | CAA1 | TTCTAATACGACTCACTATAGGTAGCAGTTCTGC ACTAGAGTTTTAGAGCTAGA |
| | CAA2 | TTCTAATACGACTCACTATAGCCAGCGGAGGTT GCGGACTCGTTTTAGAGCTAGA |
| | CAA3 | TTCTAATACGACTCACTATAGGAAAGAGATTCTG TCCATAC GTTTTAGAGCTAGA |
| | CAA4 | TTCTAATACGACTCACTATAGTCTTTATGGTGTCT TCTCTGGTTTTAGAGCTAGA |
| | CAA5 | TTCTAATACGACTCACTATAGGCCTCTCCCTCTG GAATCTGTTTTAGAGCTAGA |
| | CAA6 | TTCTAATACGACTCACTATAGTGCAGAATTCAGG ATCTACAGTTTTAGAGCTAGA |
| *bmp6b* | CAA7 | TTCTAATACGACTCACTATAGGCAGGTAGTAGTT CTGTACGTTTTAGAGCTAGA |
| | CAA8 | TTCTAATACGACTCACTATAGAGCCCAACTTCAT TCATCGGGTTTTAGAGCTAGA |
| | CAA9 | TTCTAATACGACTCACTATAGGAAAGAGATCCT GTCCATACGTTTTAGAGCTAGA |
| | CAA10 | TTCTAATACGACTCACTATAGGCCTCTCCCTCTG GAATCTGTTTTAGAGCTAGA |
| | CAA11 | TTCTAATACGACTCACTATAGAGCAGAATTCAG GATCTACAGTTTTAGAGCTAGA |

3. The reagent according to claim 1, wherein the nucleotide sequence of the sgRNA reverse primer is: 5'-

GATCCGCACCGACTCGGTGCCACTTTTTCAAGTTGATAACGGACTAGCCTTATTTTAACTTGCTATTTCT
AGCTCTAAAAC-3'.

## Patentansprüche

1. Ein Reagenz zum Ausschalten der *bmp6a* und *bmp6* Gene im Genom von *Carassius auratus,* wobei das Reagenz sgRNA-Vorwärtsprimer und sgRNA-Rückwärtsprimer umfasst, die für Knockout-Zielstellen der bmp6-Gene entwickelt wurden; wobei die Knockout-Zielstellen der bmp6-Gene in Tabelle 1 aufgeführt sind,
| Tabelle 1Gen | sgRNAs Nummer | Genomkoordinaten | Exon | Zielsequenz |
|---|---|---|---|---|
| *bmp6a* | CAA1 | NC_039266.1:578 4476 | Exon1 | GGTAGCAGTTCTGCA CTAGACGG |
| | CAA2 | NC_039266.1:578 4523 | Exon1 | CCAGCGGAGGTTGCG GACTCAGG |
| | CAA3 | NC_039266.1:578 4562 | Exon1 | GAAAGAGATTCTGTC CATACTGG |
| | CAA4 | NC_039266.1:581 5487 | Exon2 | TCTTTATGGTGTCTTCT CTGTGG |
| | CAA5 | NC_039266.1:581 5527 | Exon2 | GGCCTCTCCCTCTGGA ATCTGGG |
| | CAA6 | NC_039266.1:581 5558 | Exon2 | TGCAGAATTCAGGAT CTACAAGG |
| *bmp6b* | CAA7 | NC_039291.1:812 7537 | Exon1 | GGCAGGTAGTAGTTC TGTACTGG |
| | CAA8 | NC_039291.1:812 7477 | Exon1 | AGCCCAACTTCATTCA TCGGAGG |
| | CAA9 | NC_03929.1:8127 425 | Exon1 | GAAAGAGATCCTGTC CATACTGG |
| | CAA10 | NC_03929.1:8097 264 | Exon2 | GGCCTCTCCCTCTGGA ATCTGGG |
| | CAA11 | NC_039291.1:809 7233 | Exon2 | AGCAGAATTCAGGAT CTACAAGG |

2. Das Reagenz gemäß Anspruch 1, wobei die sgRNA-Vorwärtsprimer in der folgenden Tabelle aufgeführt sind:
| Gen | sgRNAs Nummer | sgRNA Vorwärtsprimer |
|---|---|---|
| *bmp6a* | CAA1 | TTCTAATACGACTCACTATAGGTAGCAGTTCTGC ACTAGAGTTTTAGAGCTAGA |
| | CAA2 | TTCTAATACGACTCACTATAGCCAGCGGAGGTT GCGGACTCGTTTTAGAGCTAGA |
| | CAA3 | TTCTAATACGACTCACTATAGGAAAGAGATTCTG TCCATAC GTTTTAGAGCTAGA |
| | CAA4 | TTCTAATACGACTCACTATAGTCTTTATGGTGTCT TCTCTGGTTTTAGAGCTAGA |
| | CAA5 | TTCTAATACGACTCACTATAGGCCTCTCCCTCTG GAATCTGTTTTAGAGCTAGA |
| | CAA6 | TTCTAATACGACTCACTATAGTGCAGAATTCAGG ATCTACAGTTTTAGAGCTAGA |
| *bmp6b* | CAA7 | TTCTAATACGACTCACTATAGGCAGGTAGTAGTT CTGTACGTTTTAGAGCTAGA |
| | CAA8 | TTCTAATACGACTCACTATAGAGCCCAACTTCAT TCATCGGGTTTTAGAGCTAGA |
| | CAA9 | TTCTAATACGACTCACTATAGGAAAGAGATCCT GTCCATACGTTTTAGAGCTAGA |
| | CAA10 | TTCTAATACGACTCACTATAGGCCTCTCCCTCTG GAATCTGTTTTAGAGCTAGA |
| | CAA11 | TTCTAATACGACTCACTATAGAGCAGAATTCAG GATCTACAGTTTTAGAGCTAGA |

3. Das Reagenz gemäß Anspruch 1, wobei die Nukleotidsequenz des sgRNA-Rückwärtsprimers wie folgt lautet: 5'-

GATCCGCACCGACTCGGTGCCACTTTTTCAAGTTGATAACGGACTAGCCTTATTTTAACTTGCTATTTCT
AGCTCTAAAAC-3'.

## Revendications

1. Réactif pour éliminer des gènes bmp6a et bmp6b dans le génome d'un *Carassius auratus,* le réactif comprenant des amorces sens de sgARN et des amorces antisens de sgARN conçues pour éliminer des sites cibles des gènes bmp6 ; dans lequel les sites cibles d'élimination des gènes bmp6 sont illustrés dans le tableau 1
| Tableau 1 gène | Numéro des sgARN | Coordonnées du génome | Exon | Séquence cible |
|---|---|---|---|---|
| Bmp6a | CAA1 | NC_039266.1:578 4476 | Exon1 | GGTAGCAGTTCTGCA CTAGACGG |
| | CAA2 | NC_039266.1:578 4523 | Exon1 | CCAGCGGAGGTTGCG GACTCAGG |
| | CAA3 | NC_039266.1:578 4562 | Exon1 | GAAAGAGATTCTGTC CATACTGG |
| | CAA4 | NC_039266.1:581 5487 | Exon2 | TCTTTATGGTGTCTTCT CTGTGG |
| | CAA5 | NC_039266.1:581 5527 | Exon2 | GGCCTCTCCCTCTGGA ATCTGGG |
| | CAA6 | NC_039266.1:581 5558 | Exon2 | TGCAGAATTCAGGAT CTACAAGG |
| Bmp6b | CAA7 | NC_039291.1:812 7537 | Exon1 | GGCAGGTAGTAGTTC TGTACTGG |
| | CAA8 | NC_039291.1:812 7477 | Exon1 | AGCCCAACTTCATTCA TCGGAGG |
| | CAA9 | NC_03929.1:8127 425 | Exon1 | GAAAGAGATCCTGTC CATACTGG |
| | CAA10 | NC_03929.1:8097 264 | Exon2 | GGCCTCTCCCTCTGGA ATCTGGG |
| | CAA11 | NC_039291.1:809 7233 | Exon2 | AGCAGAATTCAGGAT CTACAAGG |

2. Réactif selon la revendication 1, dans lequel les amorces sens de sgARN sont illustrées dans le tableau suivant
| Gène | Numéro du sgARN | Amorce sens de sgARN |
|---|---|---|
| Bmp6a | CAA1 | TTCTAATACGACTCACTATAGGTAGCAGTTCTGC ACTAGAGTTTTAGAGCTAGA |
| | CAA2 | TTCTAATACGACTCACTATAGCCAGCGGAGGTT GCGGACTCGTTTTAGAGCTAGA |
| | CAA3 | TTCTAATACGACTCACTATAGGAAAGAGATTCTG TCCATAC GTTTTAGAGCTAGA |
| | CAA4 | TTCTAATACGACTCACTATAGTCTTTATGGTGTCT TCTCTGGTTTTAGAGCTAGA |
| | CAA5 | TTCTAATACGACTCACTATAGGCCTCTCCCTCTG GAATCTGTTTTAGAGCTAGA |
| | CAA6 | TTCTAATACGACTCACTATAGTGCAGAATTCAGG ATCTACAGTTTTAGAGCTAGA |
| Bmp6b | CAA7 | TTCTAATACGACTCACTATAGGCAGGTAGTAGTT CTGTACGTTTTAGAGCTAGA |
| | CAA8 | TTCTAATACGACTCACTATAGAGCCCAACTTCAT TCATCGGGTTTTAGAGCTAGA |
| | CAA9 | TTCTAATACGACTCACTATAGGAAAGAGATCCT GTCCATACGTTTTAGAGCTAGA |
| | CAA10 | TTCTAATACGACTCACTATAGGCCTCTCCCTCTG GAATCTGTTTTAGAGCTAGA |
| | CAA11 | TTCTAATACGACTCACTATAGAGCAGAATTCAG GATCTACAGTTTTAGAGCTAGA |

3. Réactif selon la revendication 1, dans lequel la séquence nucléotidique de l'amorce antisens de sgARN est :
